(19) Europäisches Patentamt
European Patent Office
Office européen des brevets

(11) EP 1 483 244 B1

(12) EUROPÄISCHE PATENTSCHRIFT

(45) Veröffentlichungstag und Bekanntmachung des Hinweises auf die Patenterteilung:
**10.04.2013 Patentblatt 2013/15**

(21) Anmeldenummer: **03709711.0**

(22) Anmeldetag: **18.02.2003**

(51) Int Cl.:
***C07D 217/26*** *(2006.01)* ***C07D 471/04*** *(2006.01)*
***A61K 31/472*** *(2006.01)* ***A61K 31/437*** *(2006.01)*
***A61P 19/00*** *(2006.01)* *C07D 471/04 (2006.01)*
*C07D 235/00 (2006.01)* *C07D 221/00 (2006.01)*

(86) Internationale Anmeldenummer:
**PCT/EP2003/001596**

(87) Internationale Veröffentlichungsnummer:
**WO 2003/074489 (12.09.2003 Gazette 2003/37)**

(54) **CYCLISCHE N-SUBSTITUIERTE ALPHA-IMINOCARBONSÄUREN ZUR SELEKTIVEN INHIBIERUNG VON KOLLAGENASE**

CYCLIC N-SUBSTITUTED ALPHA IMINOCARBOXYLIC ACIDS FOR SELECTIVELY INHIBITING COLLAGENASE

ACIDES ALPHA-IMINOCARBOXYLIQUES N-SUBSTITUÉS CYCLIQUES POUR L'INHIBITION SÉLECTIVE DE LA COLLAGENASE

(84) Benannte Vertragsstaaten:
**AT BE BG CH CY CZ DE DK EE ES FI FR GB GR HU IE IT LI LU MC NL PT SE SI SK TR**

(30) Priorität: **02.03.2002 DE 10209299**

(43) Veröffentlichungstag der Anmeldung:
**08.12.2004 Patentblatt 2004/50**

(73) Patentinhaber: **Sanofi-Aventis Deutschland GmbH**
**65929 Frankfurt am Main (DE)**

(72) Erfinder:
- **STAHL, Petra**
  **60385 Frankfurt (DE)**
- **KIRSCH, Reinhard**
  **38100 Braunschweig (DE)**
- **RUF, Sven**
  **65439 Flörsheim (DE)**
- **WEHNER, Volkmar**
  **97657 Sandberg (DE)**
- **WEITHMANN, Klaus-Ulrich**
  **65719 Hofheim (DE)**

(56) Entgegenhaltungen:
**WO-A-97/18194**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 483 244 B1

**Beschreibung**

Die Erfindung betrifft die Verwendung von cyclischen N-substituierten Alpha-Iminocarbonsäuren der Formel I zur selektiven Inhibition der Kollagenase (MMP 13). Die Verbindungen der Formel I können daher zur Behandlung degenerativer Gelenkerkrankungen eingesetzt werden.

In Erkrankungen wie Osteoarthritis und Rheuma findet eine Zerstörung des Gelenkes statt, besonders bedingt durch den proteolytischen Abbau von Kollagen durch Kollagenasen. Kollagenasen gehören zur Superfamilie der Metalloproteinasen (MP) oder Matrix-Metallproteinasen (MMP). Die MMPs bilden eine Gruppe von Zn-abhängigen Enzymen, die am biologischen Abbau der extrazellulären Matrix beteiligt sind (D. Yip et al in Investigational New Drugs 17 (1999), 387-399 und Michaelides et al in Current Pharmaceutical Design 5 (1999) 787 - 819). Diese MMPs sind insbesondere fähig, fibrilläres und nicht-fibrilläres Kollagen, sowie Proteoglycane abzubauen, die beide wichtige Matrixbestandteile darstellen. MMPs sind beteiligt an Prozessen der Wundheilung, der Tumorinvasion, Metastasenwanderung sowie an Angiogenese, multipler Sklerose, Herzversagen und Atherosklerose (Michaelides S. 788; siehe oben). Insbesondere spielen sie eine wichtige Rolle beim Abbau der Gelenkmatrix in der Arthrose und der Arthritis, sei es nun die Osteoarthrose, Osteoarthritis oder die rheumatoide Arthritis.

Eine ausgewählte Subgruppe innerhalb der MMPs wird z. B. durch die Kollagenasen gebildet. Nur Kollagenasen haben die Fähigkeit, natives Kollagen, das ja eine wichtige Stützfunktion in der Matrix ausübt, abzubauen. Diese Subgruppe besteht aus der interstitiellen Kollagenase MMP-1, der Neutrophilen-Kollagenase MMP-8, sowie der erst später identifizierten MMP-13. MMP-13 konnte im Körper von gesunden, ausgewachsenen Individuen praktisch nicht entdeckt werden, es wird nur im Verlauf von Krankheiten, z. B. in Krebszellen oder Ulcera exprimiert. MMP-13 ist auch in der Gelenkmatrix von arthrotischen Menschen und Säugetieren, insbesondere bei klinisch fortgeschrittener Arthrose nachgewiesen worden, während es im Gelenkgewebe von gesunden Erwachsenen nicht vorkommt. Die Hemmung von MMP-13 durch entsprechende Inhibitoren ist deshalb besonders geeignet, um die genannten Krankheiten zu bekämpfen.

Eine Vielzahl von verschiedenen Inhibitoren der MMP's, bzw. der Kollagenasen sind bekannt (EP 0 606 046; WO94/28889; WO 96/27583). Es ist auch bekannt, dass cyclische und heterocyclische N-substituierten Alpha-Iminohydroxam- und Carbonsäuren Inhibitoren von Metalloproteinasen sind (EP 0 861 236).

Nach den ersten klinischen Studien an Menschen hat sich nun gezeigt, dass MMPs Nebenwirkungen hervorrufen. Die hauptsächlich genannten Nebenwirkungen sind muskuloskeletale Schmerzen oder Anthralgien. Der Stand der Technik besagt eindeutig, dass erwartet wird, dass selektive Inhibitoren diese genannten Nebenwirkungen reduzieren können (Yip, Seite 387, siehe oben).

Nachteil der bekannten Inhibitoren der MMPs ist daher häufig die mangelnde Spezifität der Hemmung für nur eine Klasse der MMPs. Daher hemmen die meisten MMP-Inhibitoren mehrere MMPs gleichzeitig, weil die katalytische Domäne der MMPs eine ähnliche Struktur aufweist. Demzufolge wirken die Inhibitoren in unerwünschter Weise auf viele Enzyme, auch solche mit vitaler Funktion ein (Massova I, et al., The FASEB Journal (1998) 12,1075-1095).

In der Anmeldung WO 97/18194 (EP 0 861 236) werden bereits cyclische und heterocyclische N-substituierte Alpha-Iminohydroxam und Alpha-Iminocarbonsäuren beschrieben, die eine starke inhibitorische Wirkung auf MMP-3 und MMP-8 aufweisen. Die in WO 97/18194 offenbarten Verbindungen, die in den Beispielen beschrieben werden, zeigen auch eine starke hemmende Wirkung auf MMP-13, wie durch erneute eigene Messungen festgestellt worden ist.

In dem Bestreben, wirksame Verbindungen zur Behandlung von Bindegewebs-erkrankungen zu finden, wurde nun gefunden, dass die erfindungsgemäß eingesetzten Verbindungen starke Inhibitoren der Matrix-Metalloproteinase 13 sind, während die erfindungsgemäß eingesetzten Verbindungen im wesentlichen unwirksam sind bei den MMPs 3 und 8. Diese erfindungsgemäß eingesetzten Verbindungen sind somit viel gezielter zur selektiven Behandlung der genannten Krankheiten geeignet, als die in WO 97/18194 offenbarten und in den Beispielen beschriebenen Verbindungen, die neben MMP-13 auch noch andere MMPs inhibieren. Daher ist zu erwarten, dass diese selektiven Inhibitoren bei der Behandlung der genannten Erkrankungen ein wesentlich verbessertes Nebenwirkungsspektrum aufweisen werden.

Die Erfindung betrifft daher die Verwendung von Verbindungen der Formel II

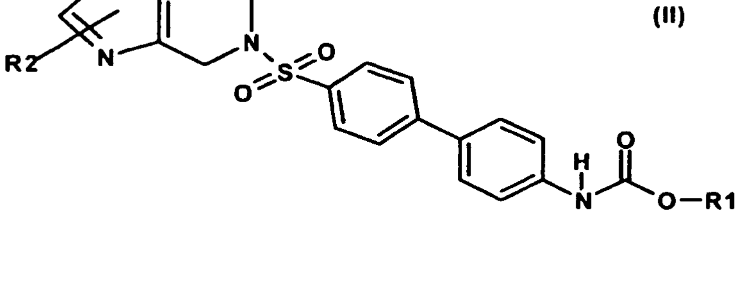

(II)

und/oder alle stereoisomeren Formen der Verbindung der Formel II und/oder Gemische dieser Formen in jedem Verhältnis, und/oder ein physiologisch verträgliches Salz der Verbindung der Formel II, wobei

R1 für 1. -($C_2$-$C_6$)-Alkyl, worin Alkyl gerade oder verzweigt ist,
2. -$CH_2$-Phenyl oder
3. -$CH_2$-Cyclopropyl, steht, und
R2 für Wasserstoffatom oder Methyl steht, oder

der Verbindung der Formel III

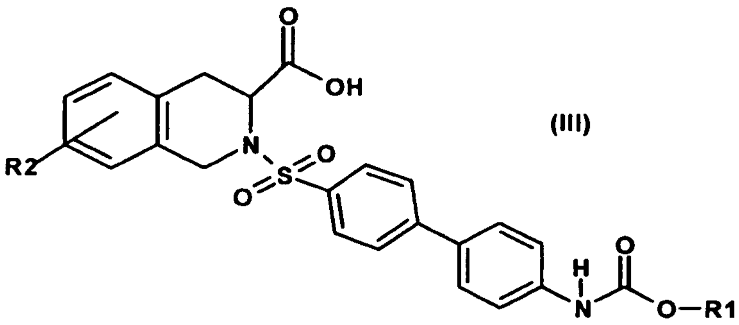

(III)

und/oder alle stereoisomeren Formen der Verbindung der Formel III und/oder Gemische dieser Formen in jedem Verhältnis, und/oder ein physiologisch verträgliches Salz der Verbindung der Formel III, wobei

R1 für 1. -($C_2$-$C_6$)-Alkyl, worin Alkyl gerade oder verzweigt ist,
2. -$CH_2$-Phenyl oder
3. -$CH_2$-Cyclopropyl, steht, und
R2 für Wasserstoffatom oder Methyl steht,

zur Herstellung eines Arzneimittels zur Prophylaxe und Therapie von Erkrankungen wie Osteoarthrosen, Spondylosen, Knorpelschwund nach Gelenktrauma oder längerer Gelenksruhigstellung nach Meniskus- oder Patellaverletzungen oder Bänderrissen, oder eine Erkrankung des Bindegewebes wie Kollagenosen, Periodontalerkrankungen, Wundheilungsstörungen, oder eine chronische Erkrankung des Bewegungsapparates wie entzündliche, immunologisch oder stoffwechselbedingte akute oder chronische Arthritiden, Arthropathien, Myalgien oder Störungen des Knochenstoffwechsels oder eine Ulceration, Atherosklerose oder Stenose oder eine entzündliche Erkrankung oder eine Krebserkrankung, Tumormetastasenbildung, Kachexie, Anorexie oder septischer Schock.

Ein weiterer Aspekt der Erfindung betrifft Verbindungen der Formel II,

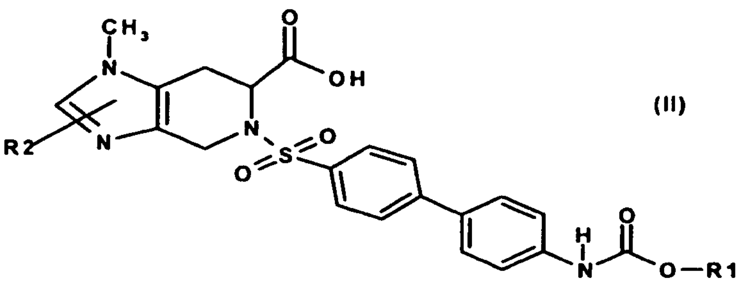

(II)

und/oder alle stereoisomeren Formen der Verbindung der Formel II und/oder Gemische dieser Formen in jedem Verhältnis, und/oder ein physiologisch verträgliches Salz der Verbindung der Formel II, wobei

R1 für 1. -($C_2$-$C_6$)-Alkyl, worin Alkyl gerade oder verzweigt ist,
2. -$CH_2$-Phenyl oder
3. -$CH_2$-Cyclopropyl, steht, und
R2 für Wasserstoffatom oder Methyl steht, oder

der Verbindungen der Formel III

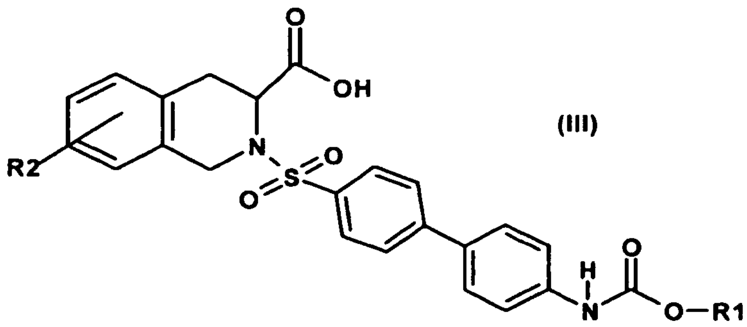

(III)

und/oder alle stereoisomeren Formen der Verbindung der Formel III und/oder Gemische dieser Formen in jedem Verhältnis, und/oder ein physiologisch verträgliches Salz der Verbindung der Formel III, wobei

R1 für 1. -($C_2$-$C_6$)-Alkyl, worin Alkyl gerade oder verzweigt ist,
2. -$CH_2$-Phenyl oder
3. -$CH_2$-Cyclopropyl, steht, und
R2 für Wasserstoffatom oder Methyl steht.

Unter dem Begriff "($C_1$-$C_{10}$)-Alkyl" werden Kohlenwasserstoffreste verstanden, deren Kohlenstoffkette geradkettig oder verzweigt ist und 1 bis 10 Kohlenstoffatome enthält, beispielsweise Methyl, Ethyl, Propyl, Iso-Propyl, Butyl, Iso-Butyl, tertiär-Butyl, Pentyl, IsoPentyl, Neopentyl, Hexyl, 2,3-Dimethylbutyl, Neohexyl, Heptyl, Octanyl, Nonanyl, Decanyl. Unter dem Begriff "($C_2$-$C_{10}$)-Alkenyl" werden Kohlenwasserstoffreste verstanden, deren Kohlenstoffketten geradkettig oder verzweigt ist und 1 bis 10 Kohlenstoffatome enthält und je nach Kettenlänge 1, 2 oder 3 Doppelbindungen enthalten. ($C_3$-$C_7$)-Cycloalkyl-Reste sind beispielsweise Verbindungen, die sich von 3- bis 7-gliedrige Monocyclen wie Cyclopropyl, Cyclobutyl, Cyclopentyl, Cyclohexyl oder Cycloheptyl herleiten.

Die Erfindung betrifft ferner ein Verfahren zur Herstellung der Verbindung der Formeln II oder III und/oder einer stereoisomeren Form der Verbindung der Formeln II oder III und/oder eines physiologisch verträglichen Salzes der Verbindung der Formeln II oder III, das dadurch gekennzeichnet ist, dass man

a) eine Verbindung der Formeln IVa oder IVb,

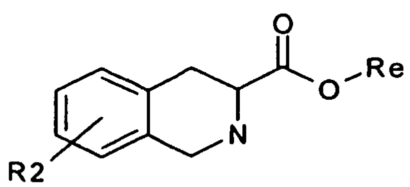

IVa

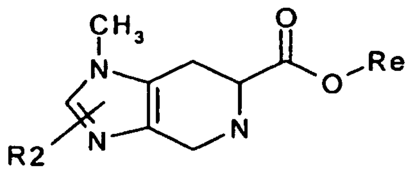

IVb

worin R2 wie in Formeln II oder III definiert ist und Re ein Wasserstoffatom oder eine Ester-Schutzgruppe darstellt, mit einer Verbindung der Formel V,

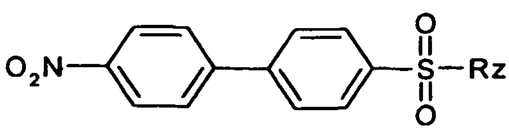

V

worin Rz Chloratom, Imidazolyl oder OH bedeutet,
in Gegenwart einer Base zu einer Verbindung der Formeln VIa oder VIb umsetzt,

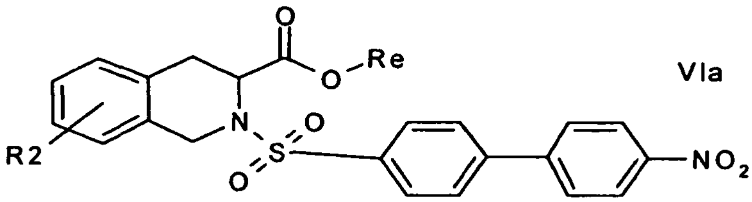

VIa

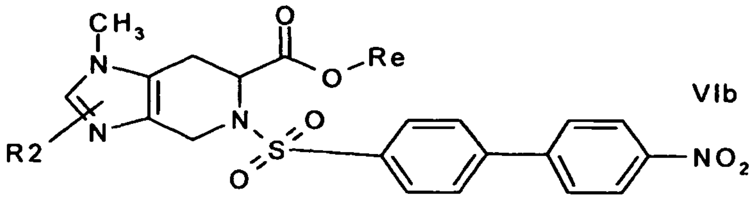

VIb

worin R2 wie in Formeln II oder III definiert ist und Re ein Wasserstoffatom oder eine Ester-Schutzgruppe darstellt, und

b) eine nach Verfahrensschritt a) hergestellte Verbindung der Formeln VIa oder VIb mit Wasserstoff und einem Metallkatalysator zu einem Amin der Formeln VIIa oder VIIb reduziert

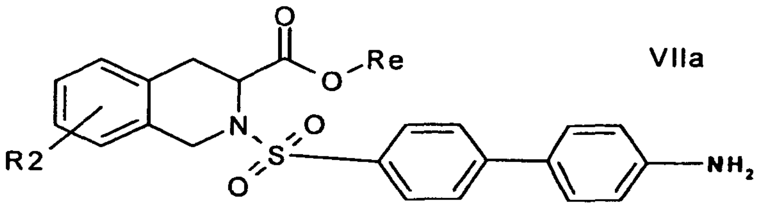

VIIa

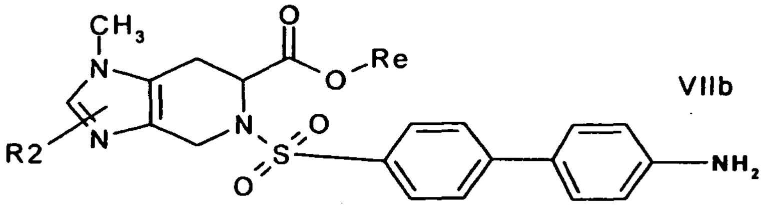

VIIb

und anschließend die Verbindung der Formeln VIIa oder VIIb mit einer Verbindung der Formel VIII

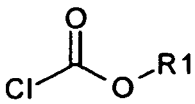

(VIII)

worin R1 wie in Formeln II oder III definiert ist, in Gegenwart einer basischen Verbindung wie Triethylamin, Trimethylamin oder Pyridin zu einer Verbindung der Formeln IXa oder IXb

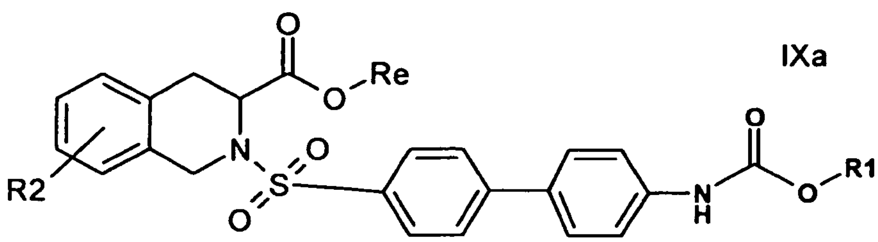

IXa

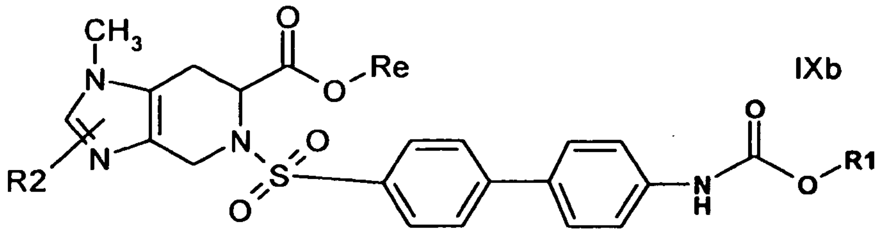

IXb

umsetzt, wobei R1 wie in Formeln II oder III definiert ist und Re wie oben definiert ist, oder

c) eine nach Verfahren a) hergestellte Verbindung der Formeln II oder III, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder

d) die nach den Verfahren b) oder c) hergestellte Verbindung der Formeln II oder III entweder in freier Form isoliert oder im Falle des Vorliegens von sauren oder basischen Gruppen in physiologisch verträgliche Salze umwandelt.

Als Ester-Schutzgruppe Re können die in Protective Groups in Organic Synthesis, T.H. Greene, P. G. M. Wuts, Wiley-Interscience, 1991, als Schutzgruppen für Ester verwendete Gruppen eingesetzt werden. Bevorzugte Ester-Schutzgruppen sind beispielsweise Methyl, Ethyl, Isopropyl, tert. Butyl oder Benzyl.

Die eingesetzten Ausgangsprodukte und Reagenzien können entweder nach bekannten Verfahren hergestellt werden oder sind käuflich erhältlich. Das Spinacinringsystem kann beispielsweise gemäß S. Klutchko et al. (J. Heterocyclic. Chem., 28, 97, (1991)) hergestellt werden.

Die Umsetzungen erfolgen beispielsweise wie in WO 97/18194 dargestellt. Die Umsetzung gemäß Verfahrensschritt a) erfolgt in Gegenwart einer Base wie KOH, NaOH, LiOH, N,O-Bis-(trimethylsilyl)acetamid (BSA), N-Methylmorpholin (NMM), N-Ethylmorpholin (NEM), Triethylamin (TEA), Diisopropylethylamin (DIPEA), Pyridin, Collidin, Imidazol oder Natriumcarbonat in Lösungsmitteln wie Tetrahydrofuran (THF), Dimethylformamid (DMF), Dimethylacetamid, Dioxan, Acetonitril, Toluol, Chloroform oder Methylenchlorid, oder auch in Gegenwart von Wasser.

Die Umsetzung gemäß Verfahrensschritt b) erfolgt beispielsweise mit den Metallkatalysatoren Pd/C, $SnCl_2$ oder Zn unter Standardbedingungen.

Im Verfahrensschritt c) wird die Verbindung der Formeln II oder III sofern sie in diastereoisomerer oder enantiomerer Form auftritt und bei der gewählten Synthese als deren Gemische anfällt, in die reinen Stereoisomeren getrennt, entweder durch Chromatographie an einem gegebenenfalls chiralen Trägermaterial, oder, sofern die razemische Verbindung der Formeln II oder III zur Salzbildung befähigt ist, durch fraktionierte Kristallisation der mit einer optisch aktiven Base oder Säure als Hilfsstoff gebildeten diastereomeren Salze. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenyl-ethylamin, Chininbasen, L-Lysin oder L- und D-Arginin die unterschiedlich löslichen diastereomeren Salze gebildet, die schwerer lösliche Komponente als Feststoff isoliert, das leichter lösliche Diastereomer aus der Mutterlauge abgeschieden, und aus den so gewonnenen diastereomeren Salzen die reinen Enantiomeren gewonnen. Auf prinzipiell gleiche Weise kann man die racemischen Verbindungen der Formeln II oder III, die eine basische Gruppe wie eine Aminogruppe enthalten, mit optisch aktiven Säuren, wie (+)-Campher-10-sulfonsäure, D- und L- Weinsäure, D-und L- Milchsäure sowie (+) und (-)-Mandelsäure in die reinen Enantiomeren überführen. Auch kann man chirale Verbindungen, die Alkohol- oder Amin-funktionen enthalten, mit entsprechend aktivierten oder gegebenenfalls N-geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführte chiralen Molekülteil mittels geeigneter Methoden wieder abspaltet.

Saure oder basische Produkte der Verbindung der Formeln II oder III können in Form ihrer Salze oder in freier Form vorliegen. Bevorzugt sind pharmakologisch verträgliche Salze, z. B. Alkali- oder Erdalkalimetallsalze bzw. Hydrochloride, Hydrobromide, Sulfate, Hemisulfate, alle möglichen Phosphate sowie Salze der Aminosäuren, natürlicher Basen oder Carbonsäuren.

Die Herstellung physiologisch verträglicher Salze aus zur Salzbildung befähigten Verbindungen der Formeln II oder III, einschließlich deren stereoisomeren Formen, gemäß Verfahrensschritt d) erfolgt in an sich bekannter Weise. Die Verbindungen der Formeln II oder III bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin oder Triethanolamin oder auch basischen Aminosäuren, etwa Lysin, Ornithin oder Arginin, stabile Alkali-, Erdalkali- oder gegebenenfalls substituierte Ammoniumsalze. Sofern die Verbindungen der Formeln II oder III basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Phosphor-, Methansulfon-, Benzolsulfon-, p-Toluolsulfon-, 4-Brombenzol-sulfon-, Cyclohexylamidosulfon-, Trifluormethylsulfon-, Essig-, Oxal-, Wein-, Bernstein- oder Trifluoressigsäure in Frage.

Die Erfindung betrifft auch Arzneimittel, gekennzeichnet durch einen wirksamen Gehalt an mindestens einer Verbindung der Formeln II oder III und/oder eines physiologisch verträglichen Salzes der Verbindung der Formeln II oder III und/oder eine gegebenenfalls stereoisomere Form der Verbindung der Formeln II oder III, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen zur selektiven Prophylaxe und Therapie all solcher Erkrankungen, an deren Verlauf eine verstärkte Aktivität der Metalloproteinase 13 beteiligt ist. Dazu gehören degenerative Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen. Ferner gehören dazu auch Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels. Ferner eignen sich die Verbindungen der Formeln II oder III zur Behandlung der Ulceration, Atherosklerose und Stenosen. Weiterhin eignen sich die Verbindungen der Formeln II oder III zur Behandlung von Entzündungen, Krebserkrankungen, Tumormetastasenbildung, Kachexie, Anorexie und septischem Schock. Die genannten Erkrankungen können mit den erfindungsgemäß eingesetzten Verbindungen wesentlich spezifischer und mit einem kleineren Nebenwirkungsspektrum eingesetzt werden, weil im wesentlichen nur MMP-13 gehemmt wird.

Die Applikation der erfindungsgemäßen Arzneimittel kann durch orale, inhalative, rektale oder transdermale Applikation oder durch subkutane, intraartikuläre, intraperitoneale oder intravenöse Injektion erfolgen. Bevorzugt ist die orale Applikation.

Die Erfindung betrifft auch ein Verfahren zur Herstellung eines Arzneimittels, das dadurch gekennzeichnet, dass man mindestens eine Verbindung der Formeln II oder III mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polyethylen-glykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formeln II oder III enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind je nach Wirksamkeit der Verbindung gemäß Formeln II oder III, Tagesdosen von etwa 20 mg bis 1000 mg Wirkstoff, bevorzugt etwa 100 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Endprodukte werden in der Regel durch massenspektroskopische Methoden (FAB-, ESI-MS) bestimmt, angegeben sind jeweils der Hauptpeak. Temperaturangaben in Grad Celsius, RT bedeutet Raumtemperatur (22 °C bis 26 °C). Verwendete Abkürzungen sind entweder erläutert oder entsprechen den üblichen Konventionen.

Nachfolgend ist die Erfindung an Hand von Beispielen näher erläutert. Herstellungsbeispiele

Die Herstellung der Verbindungen 1, 3, 4 und 5 in Tabelle 1 erfolgte analog zu den in Beispiel 2 dargestellten Verfahrensweisen.

Beispiel 2

Stufe 1: Herstellung von 2-(4'-Nitro-biphenyl-4-sulfonyl)-1,2,3,4-tetrahydro-isochinolin-3-(R)-carbonsäure

44,2 g (250 mmol) 1,2,3,4-Tetrahydroisochinolinyl-3-(R)-carbonsäure wurden in 500 ml 1N wässriger Natronlauge gelöst und unter Rühren mit 150 g (300 mmol) 4,4'-Nitrobiphenylsulfonylchlorid, gelöst in 500 ml Tetrahydrofuran, versetzt. Dabei wurde die Reaktionstemperatur auf unter 25 °C gehalten und der pH-Wert der Lösung wurde durch Zugabe von 1N NaOH auf pH 10 eingestellt. Das Reaktionsgemisch wurde 12 Stunden (h) bei Zimmertemperatur gerührt.

Zur Aufarbeitung wurde die Reaktionslösung über Celite® Klärschicht filtriert und anschließend wurde das Filtrat am Rotationsverdampfer unter reduziertem Druck auf die Hälfte des ursprünglichen Volumens eingeengt. Die erhaltene Lösung wurde mittels Zugabe von 20%iger wässriger Citronensäurelösung auf pH 2 bis 3 angesäuert, wobei das Reaktionsprodukt als farbloser Feststoff ausfiel. Der erhaltene Feststoff wurde abfiltriert und im Vakuumtrockenschrank bei 40 °C getrocknet.
Man erhielt 77,9 g (71 % der Theorie, farbloser Feststoff)
Massenspektrum: ESI: $[M + H^+]$: m/z= 439,4

Stufe 2: Herstellung von 2(4'-Nitro-biphenyl-4-sulfonyl)-1,2,3,4-tetrahydro-isochinolin-3-(R)-carbonsäuremethylester

Zu einer Lösung von 65 ml Thionylchlorid (885 mmol) in 800 ml Methanol wurden bei einer Temperatur von -15 °C bis -10 °C 77,5 g (177 mmol) der in Stufe 1 hergestellten Carbonsäure portionsweise zugegeben. Anschließend wurde 1 Stunde bei 25 °C sowie 1 Stunde bei 50 °C gerührt.

Zur Aufarbeitung wurde die Reaktionslösung unter reduziertem Druck eingeengt und mit 300 ml Dichlormethan und 300 ml gesättigter wässriger $NaHCO_3$-Lösung verrührt. Die organische Phase wurde anschließend mit Wasser neutral gewaschen, über $Na_2SO_4$ getrocknet und das Lösungsmittel unter reduziertem Druck entfernt. Das auf diese Weise erhaltene Rohprodukt (80 g, braunes Öl) wurde mittels Chromatographie an Kieselgel (40μ - 63μ) mit n-Heptan: Ethylacetat=2:1 als mobiler Phase gereinigt.
Man erhielt 27 g (34 % der Theorie (d.Th.), farbloses Öl)
Massenspektrum: ESI: $[M + H^+]$: m/z= 453,4

Stufe 3: Herstellung von 2(4'-Amino-biphenyl-4-sulfonyl)-1,2,3,4-tetrahydro-isochinolin-3-(R)-carbonsäuremethylester

53 g (117 mmol) der gemäß Stufe 2 hergestellten Nitroverbindung wurden in 1,5 l einer 1:1 Mischung aus Tetrahydrofuran (THF) und Methanol gelöst, mit 1 g Pd (10 % auf Aktivkohle) versetzt und in einer Hydrierapparatur mit $H_2$ bis zum Ende der $H_2$-Aufnahme hydriert (Wasserstoffverbrauch 7.21).

Zur Aufarbeitung wurde der Katalysator über Celite® Klärschicht filtriert und das Filtrat am Rotationsverdampfer unter reduziertem Druck eingeengt. Der ölige Rückstand wurde in Dichlormethan aufgenommen, über $Na_2SO_4$ getrocknet und das Lösungsmittel wurde unter reduziertem Druck entfernt. Das auf diese Weise erhaltene Rohprodukt wurde aus Diethylether umkristallisiert.
Man erhielt 45,7 g (93 % d. Th., farbloser Feststoff)
Massenspektrum: ESI: $[M + H^+]$: m/z= 423,4

Stufe 4: Herstellung von 2(4'-Isopropoxycarbonylamino-biphenyl-4-sulfonyl)-1,2,3,4-tetrahydro-isochinolin-3-(R)-carbonsäuremethylester

16,9 g (40 mmol) der Verbindung aus Stufe 3 wurden in 200 ml absoluten Dichlormethan gelöst und unter Rühren bei -70 °C bis -60 °C nacheinander mit 4,9 ml Pyridin (60 mmol) sowie 44 ml (44 mmol) Chlorameisensäureisopropylester versetzt. Anschließend wurde 3 h bei dieser Temperatur gerührt. Zur Aufarbeitung wurde das Reaktionsgemisch bei 0 °C mittels Zugabe von 10 ml Wasser hydrolysiert. Nach Abtrennung der organischen Phase wurde diese neutral gewaschen, mit $Na_2SO_4$ getrocknet und das Lösungsmittel wurde unter verminderten Druck am Rotationsverdampfer entfernt. Der so erhaltene Rückstand kristallisierte nach Zugabe von 100 ml n-Pentan in Form lachsfarbener Kristalle aus.
Man erhielt 20,1g (98 %, lachsfarbene Kristalle)
Massenspektrum: ESI: $[M + H^+]$: m/z= 509,5

**Stufe** 5: Herstellung von 2(4'-Isopropoxycarbonylamino-biphenyl-4-sulfonyl)-1,2,3,4-tetrahydro-isochinolin-3-(R)-carbonsäure

20 g des gemäß Stufe 4 hergestellten Carbonsäureesters (39,4 mmol) wurden in 200 ml THF gelöst und bei 25 °C mit 48 ml 1 M wässriger LiOH-Lösung versetzt. Die Reaktionslösung wurde 3 h bei 25 °C gerührt.

Anschließend wurde das Reaktionsgemisch unter verminderten Druck eingeengt, der Rückstand in 750 ml Wasser aufgenommen, über Celite® Klärschicht unter Zusatz von Aktivkohle filtriert und die Mutterlauge mittels wässriger Zitronensäure angesäuert. Das ausgefallene Reaktionsprodukt wurde filtriert und mittels Chromatographie an Kieselgel (40 μ- 63 μ) unter Verwendung von Dichlormethan : Methanol im Verhältnis 9 : 1 als mobiler Phase gereinigt.
Man erhielt 14,1 g (72 % d. Th, farbloser Feststoff)
Massenspektrum: ESI: [M + $H^+$]: m/z= 495.2

Beispiel 6

**Stufe 1:** 5-(4'-Nitro-biphenyl-4-sulfonyl)-1-methyl-4,5,6,7-tetrahydro-1H-imidazo[4,5-c]pyridine-6-carbonsäure

2 g 1-Methylspinacin (9,19 mmol) wurden in 15 ml Wasser gelöst und bei 0 °C mit 3 Äquivalenten einer 3 N NaOH-Lösung (9,19 ml) versetzt. Nach 10 Minuten (min) tropft man eine Lösung des 4'-Nitro-biphenyl-4-sulfonylchlorids (2,74g, 9,19 mmol) in Acetonitril langsam zu, nach dem Erreichen der Raumtemperatur wird der Ansatz noch für 12 h gerührt. Das Rohprodukt wurde chromatographisch gereinigt.
Man erhielt 1,9 g (47 % d. Th, farbloser Feststoff)
Massenspektrum: ESI: [M + $H^+$]: m/z= 443

**Stufe 2:** 5-(4'-Amino-biphenyl-4-sulfonyl)-1-methyl-4,5,6,7-tetrahydro-1H-imidazo[4,5-c]pyridine-6-carbonsäure

1 g (2,3 mmol) 5-(4'-Nitro-biphenyl-4-sulfonyl)-1-methyl-4,5,6,7-tetrahydro-1H-imidazo [4,5-c]pyridin-6-carbonsäure wurde in 15 ml Dimethylformamid (DMF) gelöst, mit 0,1 g Hydrierkatalysator (10% Pd auf Aktivkohle) versetzt und innerhalb von 2 h quantitativ hydriert. Nach der Entfernung des Lösungsmittels wurde das Rohprodukt chromatographisch gereinigt.
Man erhielt 0,74 g (78 % d. Th, farbloser Feststoff)
Massenspektrum: ESI: [M + $H^+$]: m/z= 413

**Stufe 3:** 5-(4'-Alkoxycarbonylamino-biphenyl-4-sulfonyl)-1-methyl-4,5,6,7-tetrahydro-1H-imidazo[4,5-c]pyridine-6-carbonsäuren

Man löste 500 mg (1.2 mmol) 5-(4'-Amino-biphenyl-4-sulfonyl)-1-methyl-4,5,6,7-tetrahydro-1H-imidazo-[4,5-c]pyridine-6-carbonsäure in 3 ml DMF, kühlte im Eisbad auf 0 °C ab und fügte 2,4 mmol Pyridin hinzu. Nach 15 min Rühren bei 0 °C erfolgte die Zugabe von 1,8 mmol Chlorameisensäureisopropylester. Die Reaktionslösung wurde anschließend noch für 2 h bei 5 Raumtemperatur gerührt. Die Aufreinigung des Rohprodukts erfolgte mit Hilfe chromatographischer Methoden.
Man erhielt 0,38 g (64 % d. Th, farbloser Feststoff)
Massenspektrum: ESI: [M + $H^+$]: m/z= 499

Die Herstellung der Verbindungen 7 bis 10 in Tabelle 1 erfolgte analog zu den in Beispiel 6 dargestellten Verfahrensweisen.

Tabelle 1 5

| Beispiel | Struktur | MS (ESI+) |
|---|---|---|
| 1 | 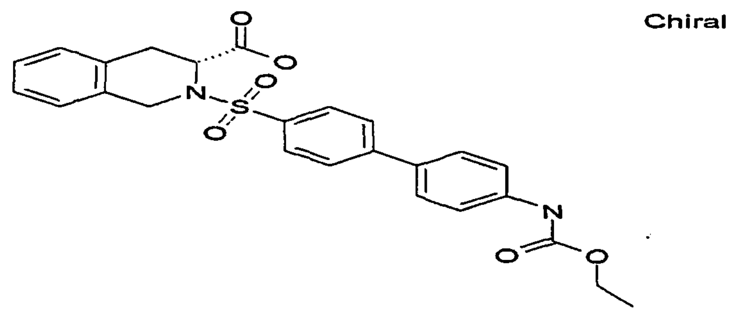 | 481 |

(fortgesetzt)

| Beispiel | Struktur | MS (ESI+) |
|---|---|---|
| 2 | 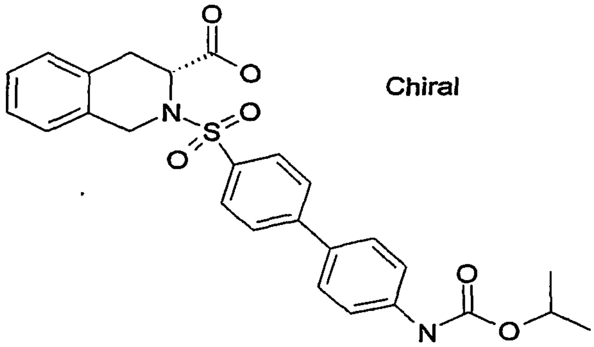 Chiral | 495 |
| 3 | 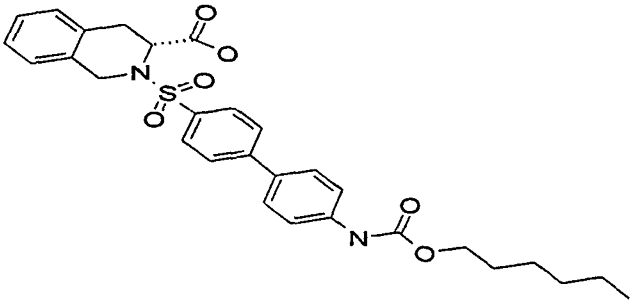 | 537 |
| 4 | 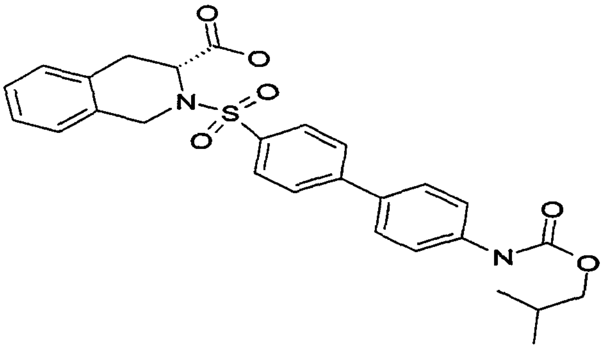 | 509 |
| 5 | 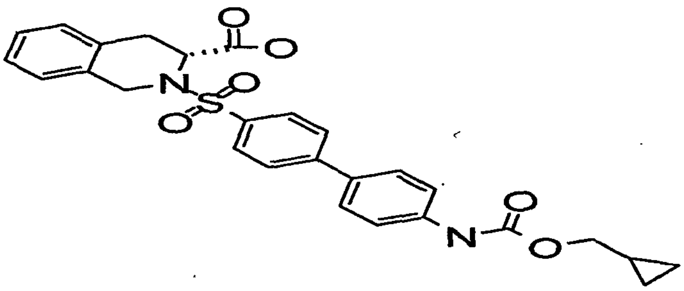 | 507 |
| 6 | 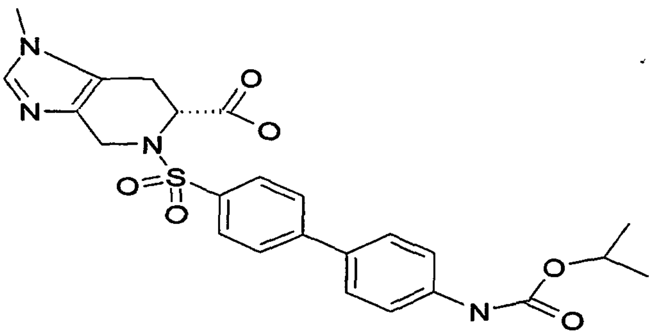 | 499 |

(fortgesetzt)

| Beispiel | Struktur | MS (ESI+) |
|---|---|---|
| 7 | 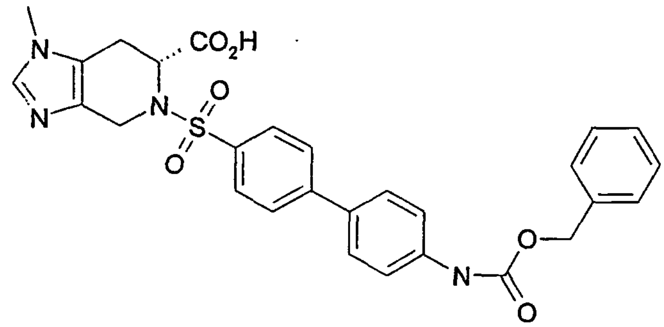 | 547 |
| 8 | 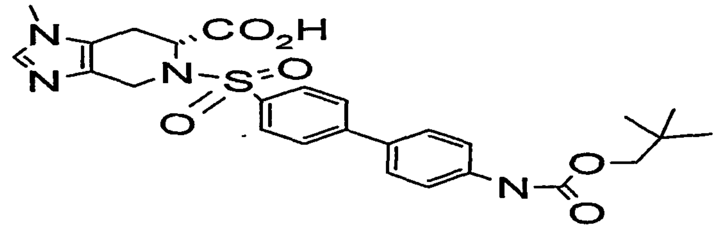 | 527 |
| 9 | 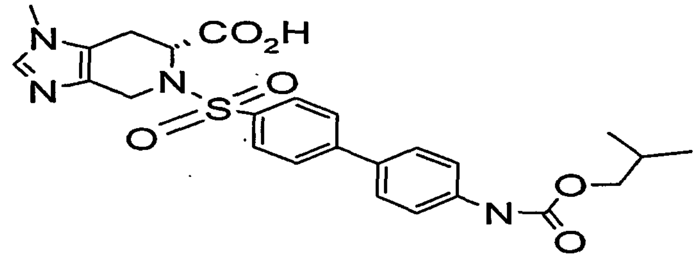 | 513 |
| 10 | 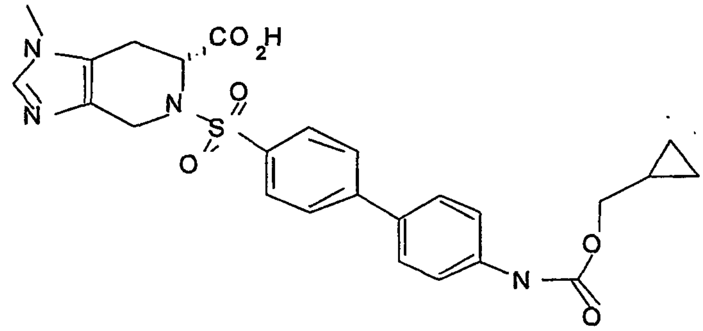 | 511 |

Pharmakologische Beispiele

Darstellung und Bestimmung der enzymatischen Aktivität der katalytischen Domäne des humanen Stromelysins (MMP 3) und der Neutrophilen-Kollagenase (MMP8).

Die beiden Enzyme -Stromelysin (MMP-3) und Neutrophilen-Kollagenase (MMP-8) - wurden dargestellt nach Ye et al. (Biochemistry; 31 (1992) Seiten 11231-11235). Zur Messung der Enzymaktivität oder der Enzyminhibitorwirkung werden bei physiologischem pH 70 µl Pufferlösung, und 10 µl Enzymlösung mit 10 µl einer 10%eigen (v/v) wässrigen Dimethylsulfoxid-Lösung, die gegebenenfalls den Enzyminhibitor enthält, für 15 Minuten inkubiert. Nach Zugabe von 10 µl einer 10%igen (v/v) wässrigen Dimethylsulfoxid-Lösung, die 1 mmol/l des Substrates enthält, wird die Enzymreaktion fluoreszenzspektroskopisch verfolgt (328 nm (ex) /393 nm(em)).

Die Enzymaktivität wird dargestellt als Extinktionszunahme/Minute. Die in Tabelle 2 aufgeführten $IC_{50}$-Werte werden als diejenige Inhibitorkonzentrationen ermittelt, die jeweils zu einer 50%igen Inhibierung des Enzyms führen.

Die Pufferlösung enthält 0,05 % Brij (Sigma, Deisenhofen, Deutschland) sowie 0,1 mol/l Tris/HCl, 0,1 mol/l NaCl, 0,01 mol/l $CaCl_2$ und 0,1 mol/l Piperazin-N,N'-bis[2-ethan-sulfonsäure] (pH=7,5).

Die Enzymlösung enthält 5 µg/ml einer der nach Ye et al. dargestellten Enzymdomänen. Die Substratlösung

enthält 1 mmol/l des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-$NH_2$ (Bachem, Heidelberg, Deutschland).

Bestimmung der enzymatischen Aktivität der katalytischen Domäne der humanen Kollagenase -3 (MMP-13).

Dieses Protein wird als inaktives Pro-Enzym von der Fa. INVITEK, Berlin, erhalten (Katalog Nr. 30100 803). Aktivierung des Proenzyms:

2 Volumenanteile Proenzym werden mit 1 Volumenanteil APMA-Lösung bei 37 °C für 1,5 Stunden inkubiert. Die APMA-Lösung wird aus einer 10 mmol/L p-Aminophenyl-Mercuric Acetate Lösung in 0,1 mmol/L NaOH durch Verdünnen mit 3 Volumenteile Tris/HCl Puffer pH7,5 (siehe unten) hergestellt. Der pH-Wert wird durch Zugabe von 1mmol/L HCl zwischen 7,0 und 7,5 eingestellt. Nach der Aktivierung des Enzyms wird dieses mit dem Tris/HCl Puffer auf eine Konzentration von 1,67 µg/mL verdünnt.

Zur Messung der Enzymaktivität werden 10 µL Enzymlösung mit 10 µL einer 3%igen (v/v) gepufferten Dimethylsulfoxid-Lösung (Reaktion 1) für 15 Minuten inkubiert. Zur Messung der Enzyminhibitoraktivität werden 10 µL Enzymlösung mit 10 µL einer 3%igen (v/v) gepufferten Dimethylsulfoxid-Lösung, die den Enzyminhibitor enthält, inkubiert (Reaktion 2).

Sowohl bei Reaktion 1 als auch bei Reaktion 2 wird nach Zugabe von 10 µL einer 3%igen (v/v) wässrigen Dimethylsulfoxid-Lösung, die 0,75 mmol/L des Substrates enthält, die Enzymreaktion fluoreszenzspektroskopisch verfolgt (328 nm (Extinktion) / 393 nm(Emission)). Die Enzymaktivität wird dargestellt als Extinktionszunahme/Minute.

Die Inhibitorwirkung wird als prozentuale Hemmung nach folgender Formel berechnet:

$$\text{\% Hemmung} = 100 - [(\text{Extinktionszunahme/Minute in Reaktion 2}) / (\text{Extinktionszunahme/Minute in Reaktion 1}) \times 100].$$

Der $IC_{50}$, d.h. die für eine 50%ige Hemmung der Enzymaktivität erforderliche Inhibitorkonzentration wird grafisch durch Auftragen der prozentualen Hemmungen bei verschiedenen Inhibitorkonzentrationen ermittelt.

Die Pufferlösung enthält 0,05% Brij (Sigma, Deisenhofen, Deutschland) sowie 0,1 mol/L Tris/HCl, 0,1 mol/L NaCl, 0,01 mol/L $CaCl_2$ (pH=7,5).

Die Enzymlösung enthält 1,67 µg/mL der Enzymdomäne.

Die Substratlösung enthält 0,75 mmol/L des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-$NH_2$ (Bachem, Heidelberg, Deutschland).

Die nachfolgende Tabelle 2 zeigt die Ergebnisse.

Tabelle 2

| Beispiel Nr. | MMP3 $IC_{50}$ (nM) | MMP 8 $IC_{50}$ (nM) | MMP 13 $IC_{50}$ (nM) |
|---|---|---|---|
| 1 | 3000 | 2300 | 40 |
| 2 | 3000 | 6000 | 30 |
| 3 | >10000 | 2000 | 70 |
| 4 | 2000. | 3000 | 20 |
| 5 | 3000 | 4000 | 20 |
| 6 | 10000 | 4000 | 80 |
| 7 | >10000 | 1000 | 60 |
| 8 | >10000 | 5000 | 70 |
| 9 | 10000 | 200 | 60 |
| 10 | >10000 | 700 | 20 |

Vergleichsbeispiele

Die folgenden in Tabelle 3 genannten Verbindungen wurden wie in WO 97/18194 beschrieben hergestellt.

Tabelle 3:

| Beispiel Nr. | Struktur | MMP 13[1)] (nM) | MMP 3[2)] (nM) | MMP 8[2)] (nM) |
|---|---|---|---|---|
| 34 | 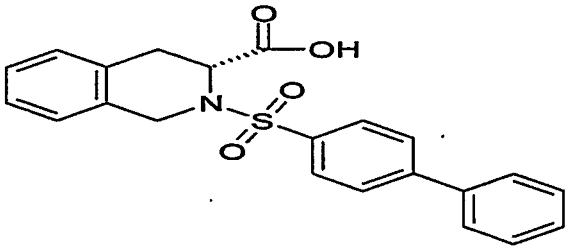 | 50 | 500 | 10 |
| 35 | 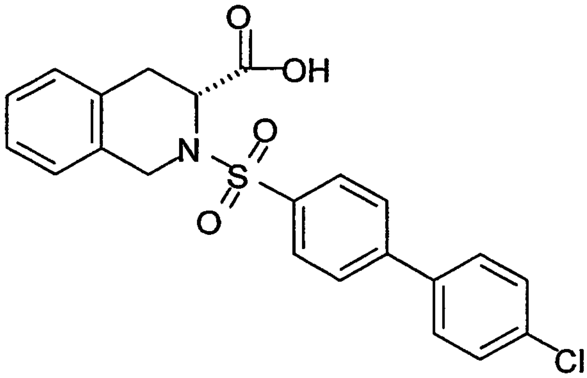 | 30 | 100 | 5 |
| 39 | 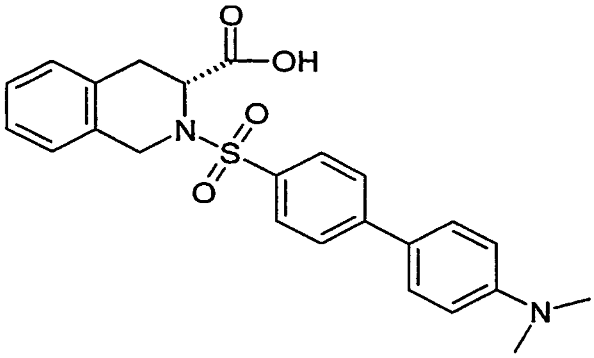 | 2,9 | 100 | 1 |
| 1) Die Bestimmung erfolgte wie oben beschrieben<br>2) und 3) Die Daten wurden der WO 97/18194 entnommen | | | | |

Die Tabelle 3 zeigt, dass strukturell ähnliche Verbindungen aus dem Stand der Technik keine Selektivität bei der Inhibition nur gegen MMP 13 aufweisen.

## Patentansprüche

1. Verwendung der Verbindung der Formel II

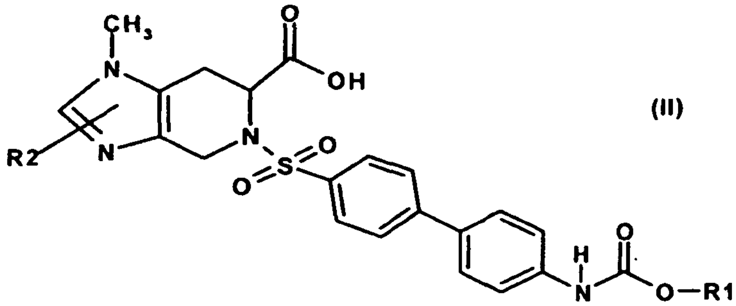

(II)

und/oder alle stereoisomeren Formen der Verbindung der Formel II und/oder Gemische dieser Formen in jedem Verhältnis, und/oder ein physiologisch verträgliches Salz der Verbindung der Formel II, wobei

R1 für 1. -($C_2$-$C_6$)-Alkyl, worin Alkyl gerade oder verzweigt ist,
2. -$CH_2$-Phenyl oder
3. -$CH_2$-Cyclopropyl, steht, und
R2 für Wasserstoffatom oder Methyl steht,

oder
der Verbindung der Formel III

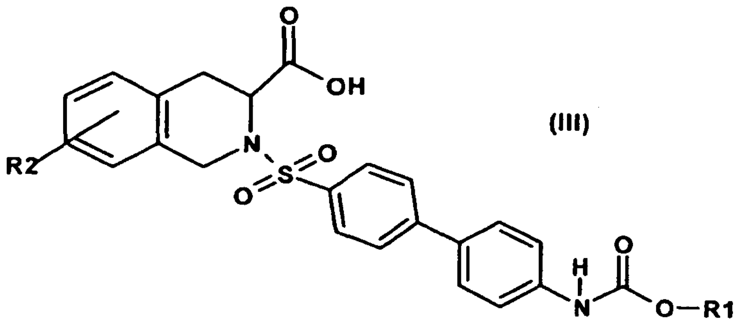

(III)

und/oder alle stereoisomeren Formen der Verbindung der Formel III und/oder Gemische dieser Formen in jedem Verhältnis, und/oder ein physiologisch verträgliches Salz der Verbindung der Formel III, wobei

R1 für 1. -($C_2$-$C_6$)-Alkyl, worin Alkyl gerade oder verzweigt ist,
2. -$CH_2$-Phenyl oder
3. -$CH_2$-Cyclopropyl, steht, und
R2 für Wasserstoffatom oder Methyl steht,

zur Herstellung eines Arzneimittels zur Prophylaxe und Therapie von Erkrankungen wie Osteoarthrosen, Spondylosen, Knorpelschwund nach Gelenktrauma oder längerer Gelenksruhigstellung nach Meniskus- oder Patellaverletzungen oder Bänderrissen, oder eine Erkrankung des Bindegewebes wie Kollagenosen, Periodontalerkrankungen, Wundheilungsstörungen, oder eine chronische Erkrankung des Bewegungsapparates wie entzündliche, immunologisch oder stoffwechselbedingte akute oder chronische Arthritiden, Arthropathien, Myalgien oder Störungen des Knochenstoffwechsels oder eine Ulceration, Atherosklerose oder Stenose oder eine entzündliche Erkrankung oder eine Krebserkrankung, Tumormetastasenbildung, Kachexie, Anorexie oder septischer Schock.

**2.** Verwendung gemäß Anspruch 1, **dadurch gekennzeichnet, dass** die Applikation durch orale, inhalative, rektale oder transdermale Gabe oder durch subkutane, intraartikuläre, intraperitonela oder intravenöse Injektion erfolgt.

**3.** Verbindung der Formel II,

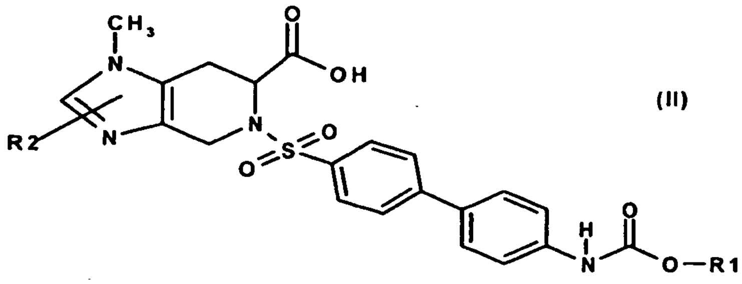

(II)

und/oder alle stereoisomeren Formen der Verbindung der Formel II und/oder Gemische dieser Formen in jedem Verhältnis, und/oder ein physiologisch verträgliches Salz der Verbindung der Formel II, wobei

R1 für 1. -($C_2$-$C_6$)-Alkyl, worin Alkyl gerade oder verzweigt ist,
2. -$CH_2$-Phenyl oder
3. -$CH_2$-Cyclopropyl, steht, und
R2 für Wasserstoffatom oder Methyl steht,

oder
der Verbindung der Formel III

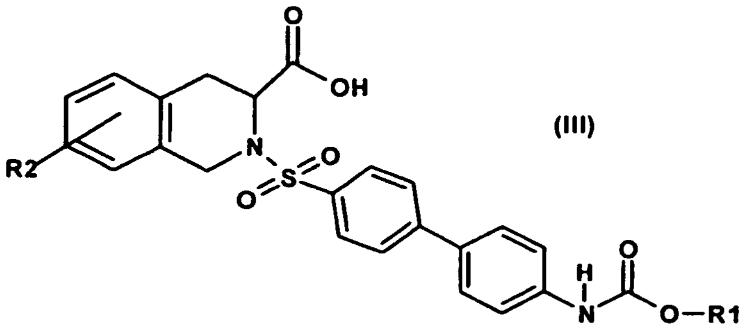

(III)

und/oder alle stereoisomeren Formen der Verbindung der Formel III und/oder Gemische dieser Formen in jedem Verhältnis, und/oder ein physiologisch verträgliches Salz der Verbindung der Formel III, wobei

R1 für 1. -($C_2$-$C_6$)-Alkyl, worin Alkyl gerade oder verzweigt ist,
2. -$CH_2$-Phenyl oder
3. -$CH_2$-Cyclopropyl, steht, und
R2 für Wasserstoffatom oder Methyl steht.

**4.** Verfahren zur Herstellung der Verbindung der Formeln II oder III gemäß Anspruch 3, **dadurch gekennzeichnet, dass** man

a) eine Verbindung der Formeln IVa oder IVb,

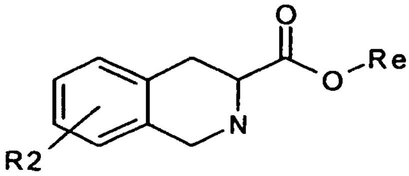

IVa

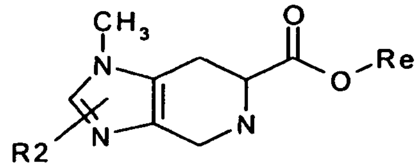

IVb

worin R2 wie in Formeln II oder III definiert ist und Re ein Wasserstoffatom oder eine Ester-Schutzgruppe darstellt, mit einer Verbindung der Formel V,

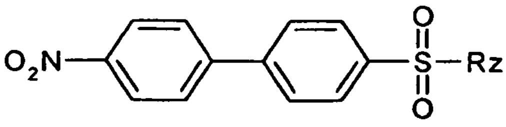

V

worin Rz Chloratom, Imidazolyl oder OH bedeutet, in Gegenwart einer Base zu einer Verbindung der Formeln VIa oder VIb umsetzt,

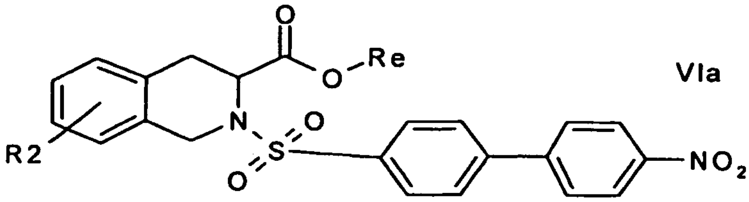

VIa

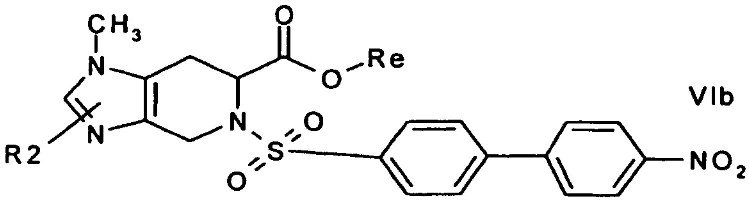

VIb

worin R2 wie in Formeln II oder III definiert ist und Re ein Wasserstoffatom oder eine Ester-Schutzgruppe darstellt, und

b) eine nach Verfahrensschritt a) hergestellte Verbindung der Formeln VIa oder VIb mit Wasserstoff und einem Metallkatalysator zu einem Amin der Formeln VIIa oder VIIb reduziert

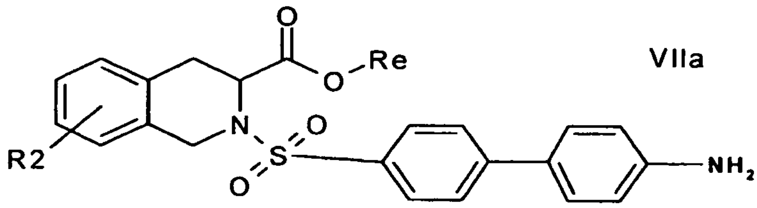

VIIa

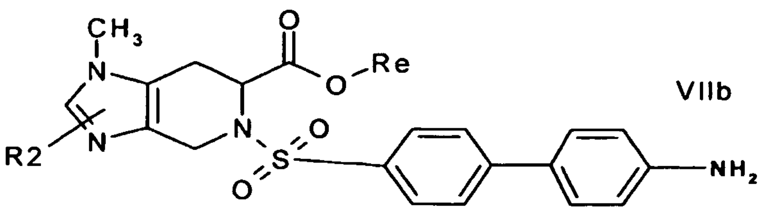

VIIb

und anschließend die Verbindung der Formeln VIIa oder VIIb mit einer Verbindung der Formel VIII

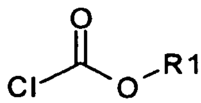

(VIII)

worin R1 wie in Formeln II oder III definiert ist, in Gegenwart einer basischen Verbindung wie Triethylamin, Trimethylamin oder Pyridin zu einer Verbindung der Formeln IXa oder

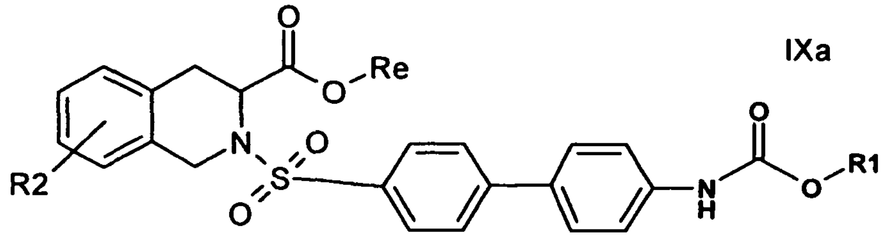

IXa

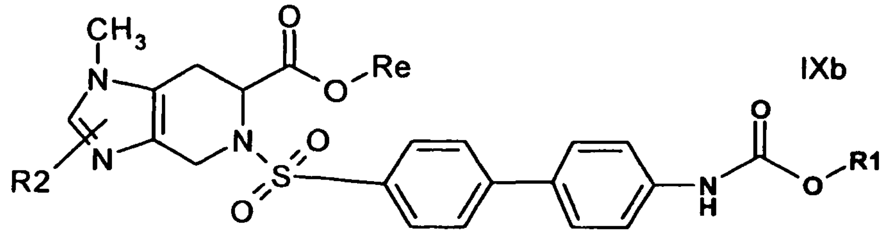

IXb

IXb

umsetzt, wobei R1 wie in Formeln II oder III definiert ist und Re wie oben definiert ist, oder

c) eine nach Verfahren a) hergestellte Verbindung der Formeln II oder III, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder

d) die nach den Verfahren b) oder c) hergestellte Verbindung der Formeln II oder III entweder in freier Form isoliert oder im Falle des Vorliegens von sauren oder basischen Gruppen in physiologisch verträgliche Salze umwandelt.

## Claims

**1.** The use of the compound of the formula II

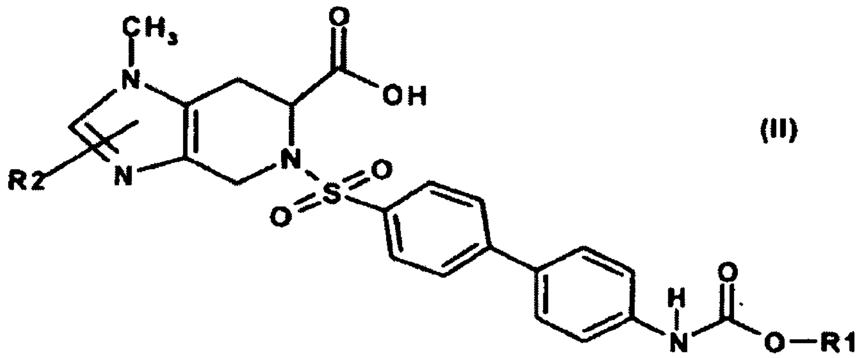

(II)

and/or all stereoisomeric forms of the compound of the formula II and/or mixtures of these forms in any ratio, and/or a physiologically tolerated salt of the compound of the formula II where

R1 is 1. -($C_2$-$C_6$)-alkyl in which alkyl is linear or branched,
2. -$CH_2$-phenyl, or
3. -$CH_2$-cyclopropyl, and
R2 is hydrogen atom or methyl,

or
of the compound of the formula III

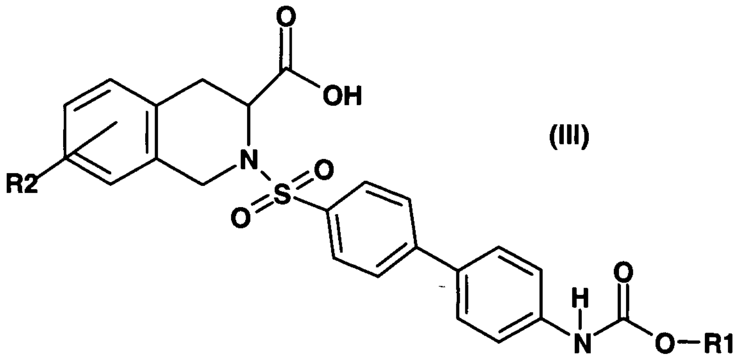

(III)

and/or all stereoisomeric forms of the compound of the formula III and/or mixtures of these forms in any ratio, and/or a physiologically tolerated salt of the compound of the formula III, where

R1 is 1. -($C_2$-$C_6$)-alkyl in which alkyl is linear or branched,
2. -$CH_2$-phenyl, or
3. -$CH_2$-cyclopropyl, and
R2 is hydrogen atom or methyl,

for producing a pharmaceutical for the prophylaxis and therapy of disorders such as osteoarthroses, spondyloses, chondrolysis after joint trauma or prolonged joint immobilization after meniscus or patella injuries or ligament tears or is a connective tissue disorder such as collagenoses, periodontal disorders, wound healing disturbances, or a chronic disorder of the locomotor system such as inflammatory, immunologically or metabolism-related acute or chronic arthritides, arthropathies, myalgias or disturbances of bone metabolism or an ulceration, atherosclerosis or stenosis or an inflammatory disorder or a cancer, tumor metastasis, cachexia, anorexia or septic shock.

2. The use as claimed in claim 1, wherein administration takes place by oral, inhalational, rectal or transdermal administration or by subcutaneous, intraarticular, intraperitoneal or intravenous injection.

3. A compound of the formula II,

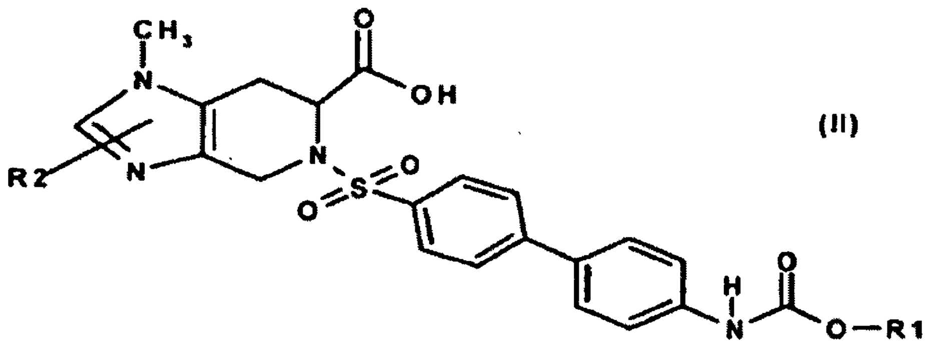

(II)

and/or all stereoisomeric forms of the compound of the formula II and/or mixtures of these forms in any ratio, and/or a physiologically tolerated salt of the compound of the formula II, where

R1 is 1. -($C_2$-$C_6$)-alkyl in which alkyl is linear or branched,
2. -$CH_2$-phenyl, or
3. -$CH_2$-cyclopropyl, and
R2 is hydrogen atom or methyl, or

of the compound of the formula III

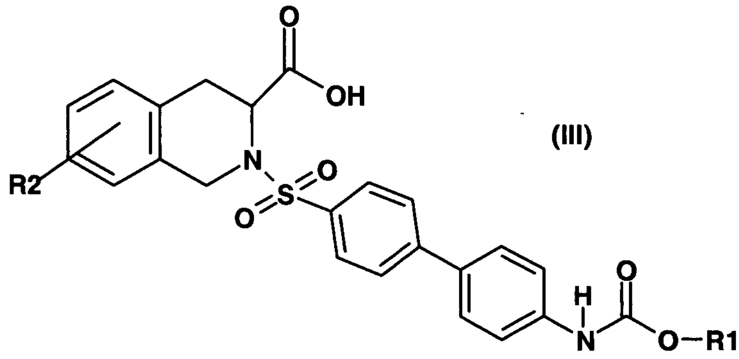

(III)

and/or all stereoisomeric forms of the compound of the formula III and/or mixtures of these forms in any ratio, and/or a physiologically tolerated salt of the compound of the formula III, where

R1 is 1. -($C_2$-$C_6$)-alkyl in which alkyl is linear or branched,

2. -$CH_2$-phenyl, or
3. -$CH_2$-cyclopropyl, and

R2 is hydrogen atom or methyl.

**4.** A process for preparing the compound of the formula II or III as claimed in claim 3, which comprises

a) reacting a compound of the formula IVa or IVb

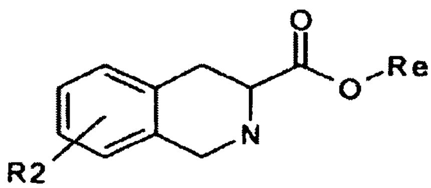

IVa

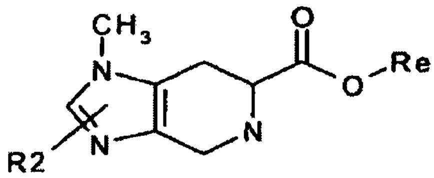

IVb

in which R2 is as defined in formula II or III and Re is a hydrogen atom or an ester protective group, with a compound of the formula V,

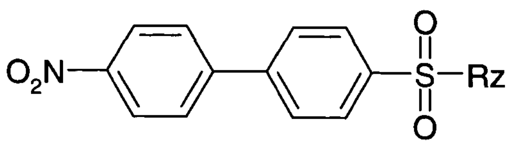

V

in which Rz is chlorine atom, imidazolyl or OH,
in the presence of a base to give a compound of the formula VIa or VIb

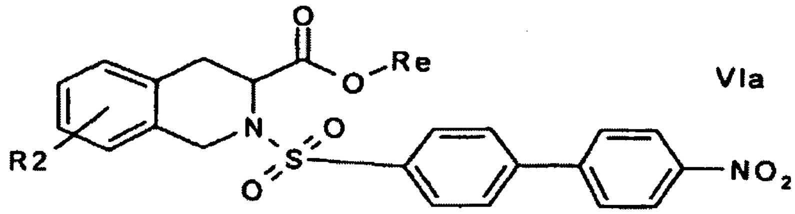

VIa

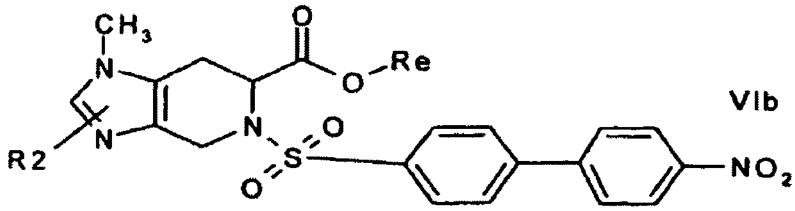

VIb

in which R2 is as defined in formula II or III and Re is a hydrogen atom or an ester protective group, and
b) reducing a compound of the formula VIa or VIb prepared as in process step a)
with hydrogen and a metal catalyst to an amine of the formula VIIa or VIIb

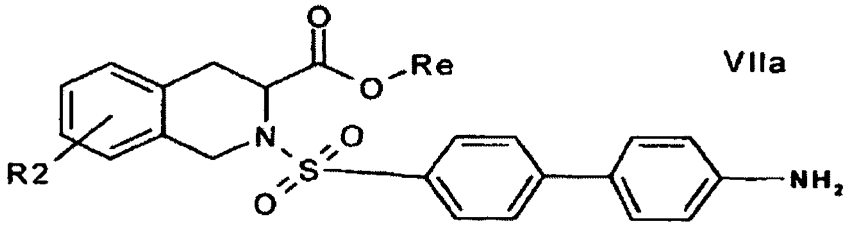

VIIa

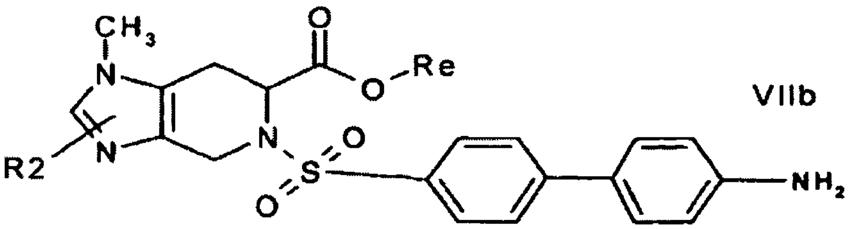

VIIb

and subsequently reacting the compound of the formula VIIa or VIIb with a compound of the formula VIII

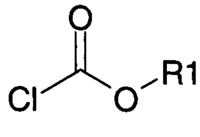

**(VIII)**

in which R1 is as defined in formula II or III,
in the presence of a basic compound such as triethylamine, trimethylamine or pyridine to give a compound of the formula IXa or IXb

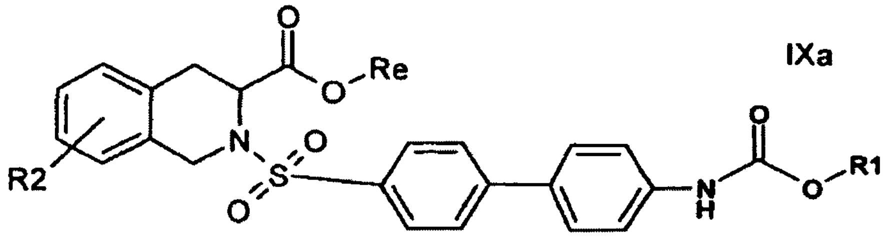

IXa

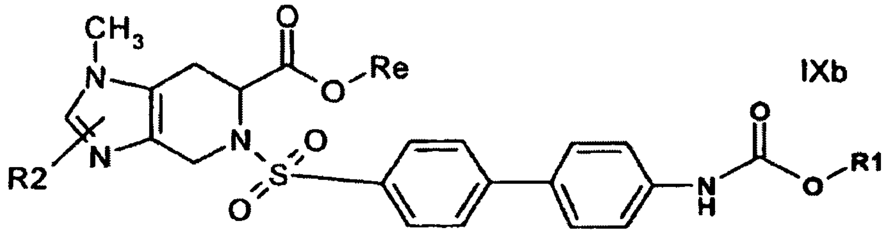

IXb

where R1 is as defined in formula II or III and Re is as defined above, or

c) fractionating a compound of the formula II or III which has been prepared by process a) and which, because of its chemical structure, occurs in enantiomeric forms into the pure enantiomers by salt formation with enantiopure acids or bases, chromatography on chiral stationary phases or derivatization using chiral enantiopure compounds such as amino acids, separation of the diastereomers obtained in this way, and elimination of the chiral auxiliary groups, or

d) either isolating in free form the compound of the formula II or III prepared by processes b) or c) or, in the case where acidic or basic groups are present, converting into physiologically tolerated salts.

## Revendications

**1.** Utilisation du composé de formule II

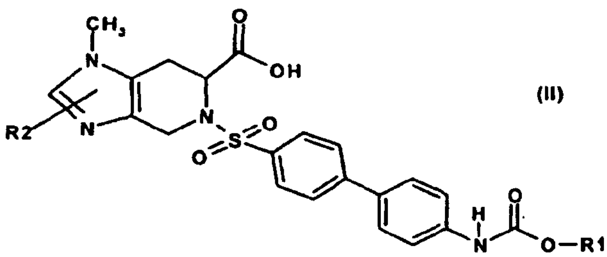

(II)

et/ou de toutes les formes stéréo-isomères du composé de formule II et/ou de tous les mélanges de ces formes, dans tous les rapports, et/ou d'un sel physiologiquement acceptable du composé de formule II, où

R1 représente
1. -($C_2$-$C_6$)-alkyle, où alkyle est linéaire ou ramifié,
2. -$CH_2$-phényle ou
3. -$CH_2$-cyclopropyle, et
R2 représente un atome d'hydrogène ou méthyle, ou

du composé de formule III

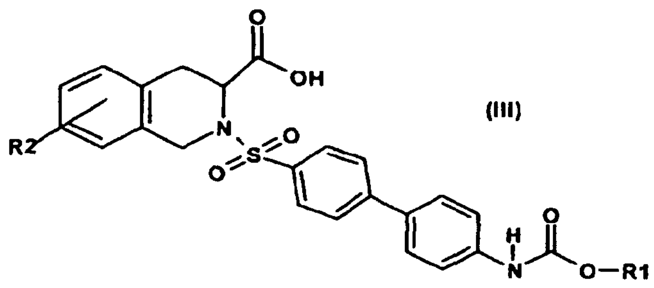

(III)

et/ou de toutes les formes stéréo-isomères du composé de formule III et/ou de tous les mélanges de ces formes, dans tous les rapports, et/ou d'un sel physiologiquement acceptable du composé de formule III, où

R1 représente
1. -($C_2$-$C_6$)-alkyle, où alkyle est linéaire ou ramifié,
2. -$CH_2$-phényle ou
3. -$CH_2$-cyclopropyle, et
R2 représente un atome d'hydrogène ou méthyle,

pour la préparation d'un médicament destiné à la prophylaxie et la thérapie de maladies telles que les ostéoarthroses, les spondyloses, les blessures au cartilage après un traumatisme articulaire ou une immobilisation prolongée des articulations après des blessures au ménisque ou à la rotule ou des déchirures des ligaments, ou une maladie du tissu conjonctif, telle que les collagénoses, les maladies parodontales, les troubles de guérison de plaies, ou une maladie chronique de l'appareil locomoteur, telle que les arthrites aiguës ou chroniques, inflammatoires, immunologiques ou provoquées par le métabolisme, les arthropathies, les myalgies ou les troubles du métabolisme osseux ou une ulcération, l'athérosclérose ou la sténose ou une maladie inflammatoire ou une maladie cancéreuse, une formation de métastases tumorales, la cachexie, l'anorexie ou le choc septique.

2. Utilisation selon la revendication 1, **caractérisée en ce que** l'administration a lieu par une administration orale, par inhalation, rectale ou transdermique ou par une injection sous-cutanée, intra-articulaire, intrapéritonéale ou intraveineuse.

3. Composé de formule II,

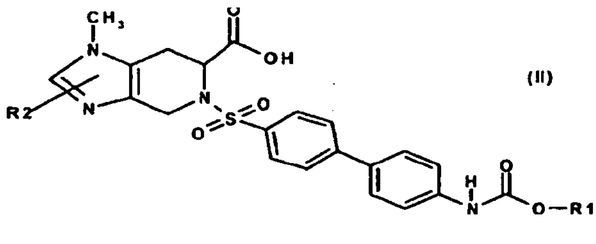

(II)

et/ou toutes les formes stéréo-isomères du composé de formule II et/ou tous les mélanges de ces formes, dans tous les rapports, et/ou un sel physiologiquement acceptable du composé de formule II, où

R1 représente
1. -($C_2$-$C_6$)-alkyle, où alkyle est linéaire ou ramifié,
2. -$CH_2$-phényle ou
3. -$CH_2$-cyclopropyle, et
R2 représente un atome d'hydrogène ou méthyle, ou

composé de formule III

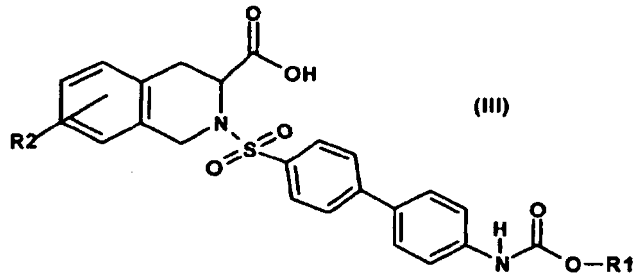

(III)

et/ou toutes les formes stéréo-isomères du composé de formule III et/ou tous les mélanges de ces formes, dans tous les rapports, et/ou un sel physiologiquement acceptable du composé de formule III, où

R1 représente
1. -($C_2$-$C_6$)-alkyle, où alkyle est linéaire ou ramifié,
2. -$CH_2$-phényle ou
3. -$CH_2$-cyclopropyle, et
R2 représente un atome d'hydrogène ou méthyle.

4. Procédé pour la préparation du composé de formule II ou III selon la revendication 3, **caractérisé en ce qu'**on

a) transforme un composé de formule IVa ou IVb

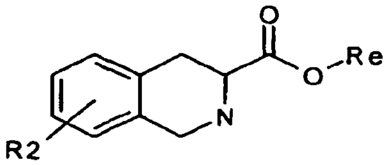

IVa

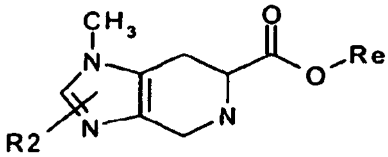

IVb

où R2 est défini comme dans la formule II ou III et Re représente un atome d'hydrogène ou un groupe de protection de la fonction ester, avec un composé de formule V,

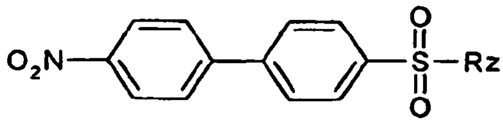

V

où Rz signifie un atome de chlore, imidazolyle ou OH,
en présence d'une base en un composé de formule VIa ou VIb,

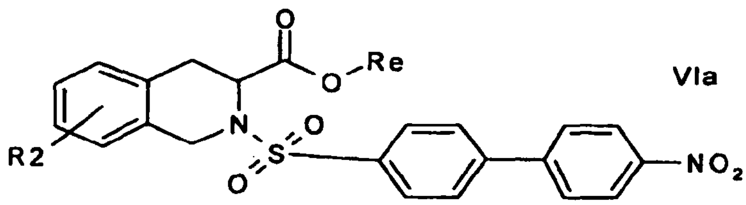

VIa

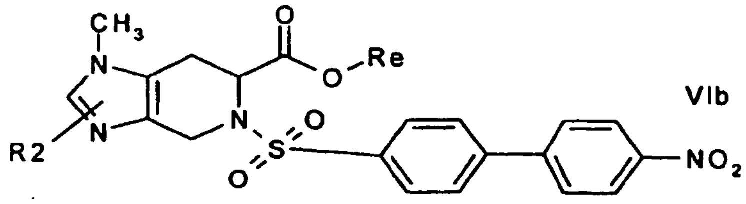

VIb

où R2 est défini comme dans la formule II ou III et Re représente un atome d'hydrogène ou un groupe de protection de la fonction ester, et

b) réduit un composé de formule VIa ou VIb préparé selon l'étape de procédé a)

avec de l'hydrogène et un catalyseur métallique en une amine de formule VIIa ou VIIb

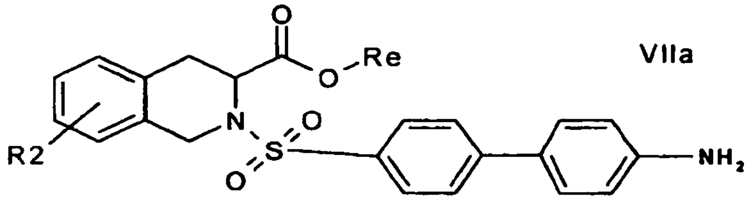

VIIa

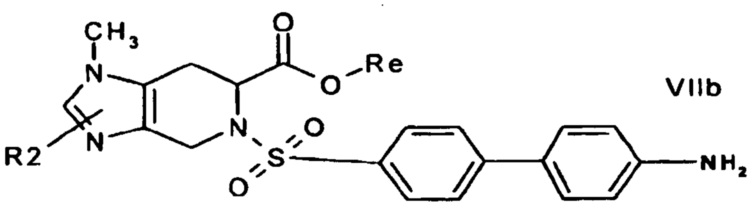

VIIb

et transforme ensuite le composé de formule VIIa ou VIIb avec un composé de formule VIII

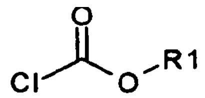

(VIII)

où R1 est défini comme dans la formule II ou III,

en présence d'un composé basique, tel que la triéthylamine, la triméthylamine ou la pyridine en un composé de formule IXa ou IXb

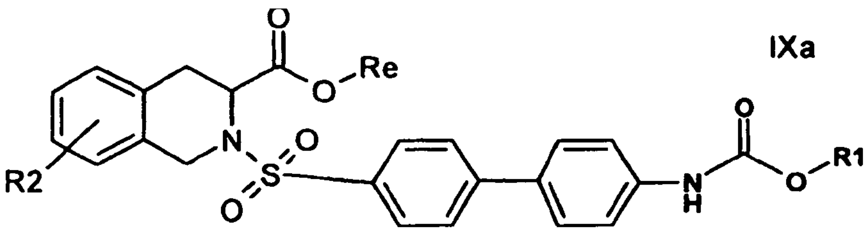

IXa

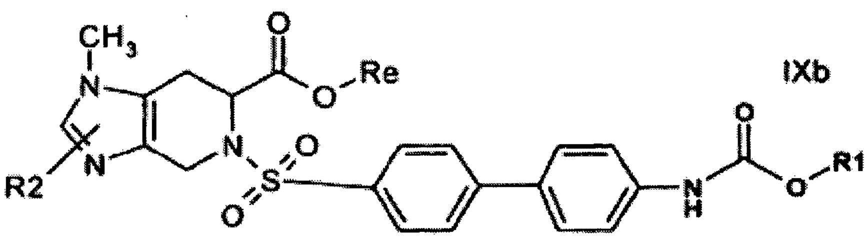

IXb

où R1 est défini comme dans la formule II ou III et Re est tel que défini ci-dessus, ou

c) sépare un composé de formule II ou III, préparé selon le procédé a), qui, en raison de sa structure chimique, apparaît sous des formes énantiomères, par salification avec des acides ou des bases sous forme d'énantiomères purs, chromatographie sur des phases stationnaires chirales ou transformation en dérivés au moyen de composés chiraux sous forme d'énantiomères purs, tels que des aminoacides, séparation des diastéréo-isomères ainsi obtenus et élimination des groupes auxiliaires chiraux, en énantiomères purs, ou

d) soit isole le composé de formule II ou III, préparé selon les procédés b) ou c) sous forme libre, soit le convertit, dans le cas de la présence de groupes acides ou basiques, en sels physiologiquement acceptables.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0606046 A
- WO 9428889 A
- WO 9627583 A
- EP 0861236 A
- WO 9718194 A


### In der Beschreibung aufgeführte Nicht-Patentliteratur


- **D. YIP et al.** *Investigational New Drugs,* 1999, vol. 17, 387-399
- **MICHAELIDES et al.** *Current Pharmaceutical Design,* 1999, vol. 5, 787-819
- **MASSOVA I et al.** *The FASEB Journal,* 1998, vol. 12, 1075-1095
- **T.H. GREENE ; P. G. M. WUTS.** Protective Groups in Organic Synthesis. Wiley-Interscience, 1991
- **S. KLUTCHKO et al.** *J. Heterocyclic. Chem.,* 1991, vol. 28, 97
- **YE et al.** *Biochemistry,* 1992, vol. 31, 11231-11235